# FASCICULE DE BREVET EUROPEEN

(11) **EP 2 114 848 B1**
(45) Date de publication et mention de la délivrance du brevet: **09.03.2011**
(21) Numéro de dépôt: 08702367.7
(22) Date de dépôt: 30.01.2008
(51) Int. Cl.: C07C 51/00, C07C 53/122

(54) **PROCEDE POUR L'OBTENTION D'ACIDE PROPIONIQUE**
VERFAHREN ZUR GEWINNUNG VON PROPIONSÄURE
METHOD FOR OBTAINING PROPIONIC ACID

(30) Priorité: 05.02.2007 FR 0700788
(43) Date de publication de la demande: 11.11.2009
(73) Titulaire: Rhodia Poliamida E Especialidades Ltda, Sao Paulo - SP (BR)
(72) Inventeur: DANILO, Zim, CEP-13013-052 Campinas, SP (BR)
(74) Mandataire: Valentino, Cédric
(86) Numéro de dépôt international: PCT/IB2008/000287
(87) Numéro de publication internationale: WO 2008/096256

(56) Documents cités:
- US-A- 2 874 187

## Description

La présente invention concerne un procédé pour la fabrication d'acide propionique par déshydratation catalytique du glycérol.

De nouvelles techniques pour la production du biodiesel se sont développées fortement au cours des dernières années. Ce carburant renouvelable diminue la pollution de l'environnement, et permet de générer des alternatives d'emplois dans des régions géographiques moins favorables à d'autres activités économiques, promouvant ainsi l'insertion et le développement social. En général, le biodiesel est produit par la transestérification de glycérides par des alcools à chaîne courte, par exemple le méthanol ou l'éthanol. Ce procédé génère du glycérol comme l'un des principaux sous-produits.

La réaction de transestérification est catalysée par un acide ou une base, selon les caractéristiques des huiles et/ou des graisses utilisées. Après la réaction de transestérification, les esters résultants sont séparés des réactifs en excès, du catalyseur et des sous-produits par un procédé comportant deux étapes. D'abord, on sépare le glycérol par décantation ou centrifugation, puis, on élimine les savons, les résidus de catalyseur et d'alcool par lavage à l'eau et barbotage ou utilisation de silicate de magnésium avec filtration.

La production importante de biodiesel s'accompagne d'une production élevée de glycérol obtenu comme sous-produit

Ainsi, il existe un besoin de développer des procédés ou applications pour valoriser le glycérol ainsi produit.

Le glycérol appelé également glycérine ou 1, 2, 3-propanetriol est un composé organique, qui contient trois fonctions hydroxyles et possède une fonction alcool. Sa structure est représentée dans la formule (I) suivante :

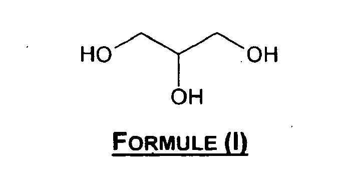

Le glycérol est utilisé notamment comme matière première pour la production de plusieurs composés. Les brevets US5387720 et US5426249 décrivent la production de 1,3-propanediol et d'acroléine à partir du glycérol. La demande de brevet US2005/0244312 décrit la production d' hydroxyacétone et de 1,2 propanediol à partir du glycérol. La demande internationale W02003035582, ainsi que la littérature (Appl. Cat. A : General 281, 2005, 225-231; Bull. Soc. Chim. France 2, 1989, 148-155; Ind. Eng. Chem. Res. 42, 2003, 2913-2923; Green Chem. 6, 2004, 359-361; J. Cat. 240, 2006, 213-221) décrivent l'utilisation de glycérol comme réactif dans des réactions d' hydrogénolyse pour la production de 1,2-propanediol et de 1,3-propanediol. Le magazine Science (nº 300, an 2003, pages 2075-2077) fait également mention de l'utilisation du glycérol comme matière première pour la production d'hydrogène et de méthane. US 2874187 décrit la production d'acide propionique à partir de ortho-crésol.

La présente invention propose une nouvelle application du glycérol consistant en l'utilisation de celui-ci comme matière première pour la fabrication de l'acide propionique.

L'acide propionique, acide propanoïque ou acide éthanocarboxylique est un acide carboxylique que l'on rencontre dans la nature. A l'état pur, c'est un liquide corrosif, incolore et qui exhale une odeur poignante. Sa structure est représentée dans la formule (II) suivante :

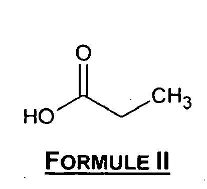

Industriellement, l'acide propionique est produit à partir de l'oxydation du propanaldéhyde avec de l'air. Les ions de cobalt ou de manganèse catalyse la réaction même à des températures élevées. En général, les procédés industriels sont conduits à une température de 40-50°C et meten en oeuvre la réaction suivante :

CH₃CH₂CHO + ½ O₂ → CH₃CH₂COOH.

De grandes quantités d'acide propionique ont déjà été produites comme sous-produits de l'acide acétique. Toutefois, les modifications des procédés de fabrication de l'acide acétique ont fortement diminués la production d'acide propionique comme sous-produit.

L'acide propionique est également produit biologiquement, à partir de la cassure métabolique des acides gras à chaînes très longues et de certains acides aminés. Des bactéries du genre *Propionibacterium,* généralement rencontrées dans les estomacs des ruminants; produisent de l'acide propionique comme produit final du métabolisme anaérobie.

L'acide propionique est par exemple utilisé dans l'inhibation de la croissance de certains champignons et de certaines bactéries. Ainsi, l'acide propionique est généralement utilisé comme agent de conservation pour les aliments destinés à la consommation humaine ou animale. L'acide propionique est également employé dans certains talcs antifongiques pour les pieds, dans la fabrication de pesticides ou de produits pharmaceutiques.

L'acide propionique peut être également utilisé comme intermédiaire chimique, par exemple, pour modifier des fibres synthétiques de cellulose.

Les dérivés de l'acide propionique possèdent également un intérêt technique et économique. Ainsi, par exemple, l'ester de l'acide propionique peut être utilisé comme solvant d'arômes artificiels.

Les nombreuses utilisations industrielles de l'acide propionique, et le nombre limité de procédés permettant son obtention, en font un produit plus avantageux que le glycérol.

L'objet de l'invention est de proposer permettant de valoriser le glycérol en acide propionique consistant à déshydrater le glycérol.

Le glycérol est avantageusement obtenu comme sous-produit des procédés de production de biodiesel. Toutefois, l'invention s'applique également à la valorisation du glycérol obtenu à partir de n'importe quelle source.

Le glycérol utilisé dans le procédé de l'invention peut être pur ou peut contenir de l'eau, par exemple entre environ 15 % et environ 90 % en poids d'eau.

La déshydratation est réalisée en une seule étape, en présence d'au moins un catalyseur comprenant un métal de transition. Ce type de catalyseur, dans les conditions de procédé de la présente invention, procure une déshydratation du glycérol, résultant en la formation de l'acide propionique.

En particulier, dans le cadre de la présente invention, les catalyseurs sont choisis parmi un ou plusieurs éléments suivants : palladium, rhodium, nickel, nickel-Raney, ruthénium et platine, éventuellement supporté sur charbon activé, silice ou alumine.

Une concentration appropriée du catalyseur peut varier entre environ 0,1 % à environ 10%, plus particulièrement environ 5% en poids.

Le rapport convenable entre la quantité de glycérol et de catalyseur (métal + support) peut varier entre environ 5 à environ 55. Un rapport convenable pour le procédé de l'invention entre les quantités de glycérol et de métal de transition contenu dans le catalyseur peut varier entre environ 10 et environ 1000.

La réaction peut être effectuée d'une manière appropriée dans des réacteurs classiques, en la présence ou non d'oxygène, particulièrement en maintenant constantes la pression et la température tout au long de la durée de la réaction.

D'une manière convenable, la température du milieu réactionnel est comprise entre environ 180°C à environ 300°C, plus particulièrement est égale à environ 250°C. La réaction est mise en oeuvre, avantageusement à une pression comprise entre environ 5 Pa à environ 4000 Pa, et est encore plus particulièrement voisine d'environ 980 Pa.

Le pH du milieu réactionnel peut avantageusement varier d'environ 2 à environ 13, particulièrement il peut être égal à environ 7.

Des exemples illustratifs de réalisations particulières de la présente invention seront donnés par la suite, sans pour autant représenter une limitation autre que celles qui sont contenues dans les revendications ci-jointes.

### Exemple 1

Un réacteur en acier inoxydable, de 150 mL de capacité, équipé d'une agitation mécanique type turbine Huston, opérant à 500 rpm, a été chargé avec 59,08 g de glycérol et 1,08 g de platine sur charbon activé comme catalyseur (Pt/C 5%, humidité de 55,31%). Le réacteur a été alors fermé, purgé à l'azote et chauffé jusqu'à 180°C sous agitation. Au fur et à mesure que la réaction se produisait, la pression relative dans le réacteur a été maintenue à 4,9 Pa.

Les produits de réaction ont été collectés au travers de la soupape de sortie des gaz, équipé d'un condenseur suivi d'un piège à 0°C (glace) et d'un piège à -69°C (glace sèche). Une purge après le dernier piège assurait la sortie des gaz incondensables.

Les produits de réaction ont été caractérisés par chromatographie gazeuse couplée avec spectrométrie de masse et quantifiés par chromatographie gazeuse avec détecteur d'ionisation de flamme en utilisant une méthode de standardisation externe. Les résultats ont été exprimés en pourcentage d'acide propionique présent dans la totalité des composés organiques condensés dans le courant de sortie du réacteur, sans tenir compte de l'eau présente dans la matière première ou éventuellement formée durant le procédé. A la fin du procédé, 49% de glycérol avaient été convertis et la concentration d'acide propionique dans le flux de sortie par rapport aux autres composés organiques a été de 0,98%.

### Exemple 2

Un réacteur en acier inoxydable, de 150 mL de capacité, équipé d'une agitation mécanique type turbine Huston, opérant à 500 rpm, a été chargé avec 59,03 g de glycérol et 10,84 g de platine sur charbon activé comme catalyseur (Pt/C 5%; humidité de 55.31%). Le réacteur a été alors fermé, purgé à l'azote et chauffé jusqu'à 250°C sous agitation. Au fur et à mesure que la réaction se produisait, la pression relative dans le réacteur a été maintenue à 980,66 Pa.

Les produits de réaction ont été collectés au travers de la soupape de sortie des gaz, équipé d'un condenseur suivi d'un piège à 0°C (glace) et d'un piège à -69°C (glace sèche). Une purge après le dernier piège assurait la sortie des gaz incondensables.

Les produits de réaction ont été caractérisés par chromatographie gazeuse couplée avec spectrométrie de masses et quantifiés par chromatographie gazeuse avec détecteur d'ionisation de flamme en utilisant une méthode de standardisation externe. Les résultats ont été exprimés en pourcentage d'acide propionique présent dans la totalité des composés organiques condensés dans le flux de sortie du réacteur, sans tenir compte de l'eau présente dans la matière première ou éventuellement formée durant le procédé. A la fin du procédé, 90% de glycérol avaient été convertis, et la concentration d'acide propionique dans le courant de sortie par rapport aux autres composés organiques a été de 1,19%.

### Exemple 3

Un réacteur en acier inoxydable, de 150 mL de capacité, équipé d'une agitation mécanique type turbine Huston, opérant à 500 rpm, a été chargé avec 59,11 g de glycérol et 1,20 g de platine sur charbon activé comme catalyseur (Pt/C 5%, humidité de 55,31 %). Le réacteur a été alors fermé, purgé à l'azote et chauffé jusqu'à 250°C sous agitation. Au fur et à mesure que la réaction se produisait, la pression relative dans le réacteur a été maintenue à 980,665 Pa.

Les produits de réaction ont été collectés au travers de la soupape de sortie des gaz, équipée d'un condenseur suivi d'un piège à 0°C (glace) et un piège à -69°C (glace sèche). Une purge après le dernier piège assurait la sortie des gaz incondensables.

Les produits de réaction ont été caractérisés par chromatographie gazeuse couplée avec spectrométrie de masses et quantifiés par chromatographie gazeuse avec détecteur d'ionisation de flamme en utilisant une méthode de standardisation externe. Les résultats ont été exprimés en pourcentage d'acide propionique présent dans la totalité des composés organiques condensés dans le flux de sortie du réacteur, sans tenir compte de l'eau présente dans la matière première ou éventuellement formée durant le procédé. A la fin du procédé, l'on a obtenu 100% de glycérol converti et la concentration d'acide propionique dans le courant de sortie par rapport aux autres composés organiques a été de 0,82%.

### Exemple 4

Un réacteur en acier inoxydable, de 150 mL de capacité, équipé d'une agitation mécanique type turbine Huston, opérant à 500 rpm, a été chargé avec 59,99 g de glycérol et 1,37 g de platine sur charbon activé comme catalyseur (Pt/C 5%, humidité 55,31%). Le pH du milieu a été ajusté avec une solution de NaOH 50% jusqu'à 13. Le réacteur a été alors fermé, purgé à l'azote et chauffé jusqu'à 210°C sous agitation. Au fur et à mesure que la réaction se produisait, la pression relative dans le réacteur a été maintenue à 980,665 Pa.

Les produits de réaction ont été collectés au travers de la soupape de sortie des gaz, équipée d'un condenseur suivi d'un piège à 0°C (glace) et un piège à -69°C (glace sèche). Une purge après le dernier piège assurait la sortie des gaz incondensables.

Les produits de réaction ont été caractérisés par chromatographie gazeuse couplée avec spectrométrie de masses et quantifiés par chromatographie gazeuse avec détecteur d'ionisation de flamme en utilisant une méthode de standardisation externe. Les résultats ont été exprimés en pourcentage d'acide propionique présent dans la totalité des composés organiques condensés dans le flux de sortie du réacteur, sans tenir compte de l'eau présente dans la matière première ou éventuellement formée durant le procédé. A la fin du procédé, l'on a obtenu 100% de glycérol converti et la concentration d'acide propionique dans le courant de sortie par rapport aux autres composés organiques a été de 0,58%.

### Exemple 5

Un réacteur en acier inoxydable, de 150 mL de capacité, équipé d'une agitation mécanique type turbine Huston, opérant à 500 rpm, a été chargé avec 58,77 g de glycérol et 1,20 g de platine sur charbon activé comme catalyseur (Pt/C 5%, humidité de 55,31%). Le réacteur a été alors fermé, purgé à l'azote et chauffé jusqu'à 300°C sous agitation. Au fur et à mesure que la réaction se produisait, la pression relative dans le réacteur a été maintenue à 980,665 Pa.

Les produits de réaction ont été collectés au travers de la soupape de sortie des gaz, équipée d'un condenseur suivi d'un piège à 0°C (glace) et un piège à -69°C (glace sèche). Une purge après le dernier piège assurait la sortie des gaz incondensables.

Les produits de réaction ont été caractérisés par chromatographie gazeuse couplée avec spectrométrie de masses et quantifiés par chromatographie gazeuse avec détecteur d'ionisation de flamme en utilisant une méthode de standardisation externe. Les résultats ont été exprimés en pourcentage d'acide propionique présent dans la totalité des composés organiques condensés dans le flux de sortie du réacteur, sans tenir compte de l'eau présente dans la matière première ou éventuellement formée durant le procédé. A la fin du procédé, l'on a obtenu une conversion de glycérol de 100% et la concentration d'acide propionique dans le courant de sortie par rapport aux autres composés organiques a été de 0,87%.

### Exemple 6

Un réacteur en acier inoxydable, de 150 mL, de capacité équipé d'une agitation mécanique type turbine Huston, opérant à 500 rpm, a été chargé avec 57,11 g de glycérol et 6,0 g de platiné sur charbon activé comme catalyseur (Pt/C 5%, humidité de 55,31%). Le réacteur a été alors fermé, purgé à l'azote et chauffé jusqu'à 250°C sous agitation. Au fur et à mesure que la réaction se produisait, la pression relative dans le réacteur a été maintenue à 980,665 Pa.

Les produits de réaction ont été récoltés au travers de la soupape de sortie des gaz, équipée d'un condenseur suivi d'un piège à 0°C (glace) et un piège à -69°C (glace sèche). Une purge après le dernier piège assurait la sortie des gaz incondensables.

Les produits de réaction ont été caractérisés par chromatographie gazeuse couplée avec spectrométrie de masses et quantifiés par chromatographie gazeuse avec détecteur d'ionisation de flamme en utilisant une méthode de standardisation externe. Les résultats ont été exprimés en pourcentage d'acide propionique présent dans la totalité des composés organiques condensés dans le flux de sortie du réacteur, sans tenir compte de l'eau présente dans la matière première ou éventuellement formée durant le procédé. A la fin du procédé, l'on a obtenu 100% de glycérol converti et la concentration d'acide propionique dans le courant de sortie par rapport aux autres composés organiques a été de 16,22%.

## Revendications

1. Procédé de fabrication d'acide propionique, **caractérisé en ce qu'**il comprend une étape de déshydratation du glycérol en présence d'au moins un catalyseur comprenant un métal de transition.

2. Procédé de fabrication d'acide propionique, selon la revendication 1, **caractérisé en ce que** le catalyseur est constitué d'un ou plusieurs éléments choisis dans le groupe comprenant le palladium, le rhodium, le nickel, le nickel-Raney, le ruthénium et le platine.

3. Procédé de fabrication d'acide propionique, selon l'une des revendications 1 ou 2, **caractérisé en ce que** le catalyseur est supporté sur un support choisi dans le groupe comprenant le charbon activé, la silice ou l'alumine

4. Procédé de fabrication d'acide propionique, selon l'une des revendications 1 à 3, **caractérisé en ce que** la concentration du catalyseur dans le milieu réactionnel est comprise entre 0,1 et 10% en poids.

5. Procédé de fabrication d'acide propionique, selon la revendication 4, **caractérisé en ce que** la concentration du catalyseur est égale à 5% en poids.

6. Procédé de fabrication d'acide propionique, selon l'une des revendications 1 a 5, **caractérisé en ce que** le rapport entre les quantités de glycérol et de catalyseur (métal + support) est compris entre environ 5 et environ 55, et le rapport entre les quantités de glycérol de métal de transition contenu dans le catalyseur est compris entre 10 et 1000.

7. Procédé de fabrication d'acide propionique, selon l'une des revendications 1 a 6, **caractérisé en ce que** le glycérol contient environ 15 à environ 90% en poids d'eau.

8. Procédé de fabrication d'acide propionique, selon l'une des revendications 1 a 7, **caractérisé en ce que** la pression et la température de la réaction sont maintenues constantes pendant la durée de la réaction.

9. Procédé de fabrication d'acide propionique, selon l'une des revendications 1 a 8, **caractérisé en ce que** la température de la réaction est comprise entre 180°C et 300°C.

10. Procédé de fabrication d'acide propionique; selon la revendication 9, **caractérisé en ce que** la température de la réaction est égale à 250°C.

11. Procédé de fabrication d'acide propionique, selon l'une des revendications 1 a 8, **caractérisé en ce que** la pression de la réaction est comprise entre 5 et 4000 Pa.

12. Procédé de fabrication d'acide propionique, selon la revendication 11, **caractérisé en ce que** la pression de la réaction est égale à 980 Pa.

13. Procédé de fabrication d'acide propionique, selon l'une des revendications 1 à 11, **caractérisé en que** le pH du milieu réactionnel est compris entre 2 et 13.

14. Procédé de fabrication d'acide propionique, selon la revendication 13, **caractérisé en ce que** le pH du milieu réactionnel est égal à 7.

## Claims

1. Method for manufacturing propionic acid, **characterized in that** it comprises a step of dehydrating glycerol in the presence of at least one catalyst comprising a transition metal.

2. Method for manufacturing propionic acid, according to Claim 1, **characterized in that** the catalyst is composed of one or more elements chosen from the group comprising palladium, rhodium, nickel, Raney nickel, ruthenium and platinum.

3. Method for manufacturing propionic acid, according to either of Claims 1 and 2, **characterized in that** the catalyst is supported on a support chosen from the group comprising activated carbon, silica or alumina.

4. Method for manufacturing propionic acid, according to one of Claims 1 to 3, **characterized in that** the concentration of the catalyst in the reaction medium is between 0.1 and 10% by weight.

5. Method for manufacturing propionic acid, according to Claim 4, **characterized in that** the concentration of the catalyst is equal to 5% by weight.

6. Method for manufacturing propionic acid, according to one of Claims 1 to 5, **characterized in that** the ratio between the amounts of glycerol and of catalyst (metal + support) is between about 5 and about 55, and the ratio between the amounts of glycerol and of transition metal contained in the catalyst is between 10 and 1000.

7. Method for manufacturing propionic acid, according to one of Claims 1 to 6, **characterized in that** the glycerol contains about 15 to about 90% by weight of water.

8. Method for manufacturing propionic acid, according to one of Claims 1 to 7, **characterized in that** the pressure and temperature of the reaction are kept constant throughout the duration of the reaction.

9. Method for manufacturing propionic acid, according to one of Claims 1 to 8, **characterized in that** the temperature of the reaction is between 180°C and 300°C.

10. Method for manufacturing propionic acid, according to Claim 9, **characterized in that** the temperature of the reaction is equal to 250°C.

11. Method for manufacturing propionic acid, according to one of Claims 1 to 8, **characterized in that** the pressure of the reaction is between 5 and 4000 Pa.

12. Method for manufacturing propionic acid, according to Claim 11, **characterized in that** the pressure of the reaction is equal to 980 Pa.

13. Method for manufacturing propionic acid, according to one of Claims 1 to 11, **characterized in that** the pH of the reaction medium is between 2 and 13.

14. Method for manufacturing propionic acid, according to Claim 13, **characterized in that** the pH of the reaction medium is equal to 7.

## Patentansprüche

1. Verfahren zur Herstellung von Propionsäure, **dadurch gekennzeichnet, daß** man Glycerin in Gegenwart mindestens eines Übergangsmetallkatalysators dehydratisiert.

2. Verfahren zur Herstellung von Propionsäure nach Anspruch 1, **dadurch gekennzeichnet, daß** der Katalysator aus einem oder mehreren Elementen aus der Gruppe bestehend aus Palladium, Rhodium, Nickel, Raney-Nickel, Ruthenium und Platin besteht.

3. Verfahren zur Herstellung von Propionsäure nach Anspruch 1 oder 2, **dadurch gekennzeichnet, daß** der Katalysator auf einem Träger aus der Gruppe bestehend aus Aktivkohle, Siliciumdioxid oder Aluminiumoxid geträgert ist.

4. Verfahren zur Herstellung von Propionsäure nach einem der Ansprüche 1 bis 3, **dadurch gekennzeichnet, daß** die Konzentration des Katalysators im Reaktionsmedium zwischen 0,1 und 10 Gew.-% liegt.

5. Verfahren zur Herstellung von Propionsäure nach Anspruch 4, **dadurch gekennzeichnet, daß** Konzentration des Katalysators 5 Gew-% beträgt.

6. Verfahren zur Herstellung von Propionsäure nach einem der Ansprüche 1 bis 5, **dadurch gekennzeichnet, daß** das Verhältnis zwischen den Mengen von Glycerin und Katalysator (Metall + Träger) zwischen ungefähr 5 und ungefähr 55 liegt und das Verhältnis zwischen den Mengen von Glycerin und im Katalysator enthaltenem Übergangsmetall zwischen 10 und 1000 liegt.

7. Verfahren zur Herstellung von Propionsäure nach einem der Ansprüche 1 bis 6, **dadurch gekennzeichnet, daß** das Glycerin ungefähr 15 bis ungefähr 90 Gew.-% Wasser enthält.

8. Verfahren zur Herstellung von Propionsäure nach einem der Ansprüche 1 bis 7, **dadurch gekennzeichnet, daß** man den Reaktionsdruck und die Reaktionstemperatur über die Reaktionsdauer konstant hält.

9. Verfahren zur Herstellung von Propionsäure nach einem der Ansprüche 1 bis 8, **dadurch gekennzeichnet, daß** die Reaktionstemperatur zwischen 180°C und 300°C liegt.

10. Verfahren zur Herstellung von Propionsäure nach Anspruch 9, **dadurch gekennzeichnet, daß** die Reaktionstemperatur 250°C beträgt.

11. Verfahren zur Herstellung von Propionsäure nach einem der Ansprüche 1 bis 8, **dadurch gekennzeichnet, daß** der Reaktionsdruck zwischen 5 und 4000 Pa liegt.

12. Verfahren zur Herstellung von Propionsäure nach Anspruch 11, **dadurch gekennzeichnet, daß** der Reaktionsdruck 980 Pa beträgt.

13. Verfahren zur Herstellung von Propionsäure nach einem der Ansprüche 1 bis 11, **dadurch gekennzeichnet, daß** der pH-Wert des Reaktionsmediums zwischen 2 und 13 liegt.

14. Verfahren zur Herstellung von Propionsäure nach Anspruch 13, **dadurch gekennzeichnet, daß** der pH-Wert des Reaktionsmediums 7 beträgt.
